# EUROPÄISCHE PATENTSCHRIFT

(11) **EP 0 729 318 B1**
(45) Veröffentlichungstag und Bekanntmachung des Hinweises auf die Patenterteilung: **07.05.1997**
(21) Anmeldenummer: 94929522.4
(22) Anmeldetag: 08.10.1994
(51) Int. Cl.: A61B 5/055, A61N 2/00

(54) **EINRICHTUNG ZUR ERMITTLUNG DER WIRKUNG GEPULSTER MAGNETFELDER AUF EINEN ORGANISMUS**
DEVICE FOR DETERMINING THE EFFECT OF PULSED MAGNETIC FIELDS ON AN ORGANISM
DISPOSITIF POUR LA DETERMINATION DE L'EFFET EXERCE PAR DES CHAMPS MAGNETIQUES PULSES SUR UN ORGANISME

(30) Priorität: 14.10.1993 DE 4335102
(43) Veröffentlichungstag der Anmeldung: 04.09.1996
(73) Patentinhaber: DR. FISCHER AG, FL-9490 Vaduz (LI)
(72) Erfinder: FISCHER, Gerhard, FL-9490 Vaduz (LI); WARNKE, Ulrich, D-66133 Scheidt (DE)
(74) Vertreter: Engelhardt, Guido, Dipl.-Ing.
(86) Internationale Anmeldenummer: EP9403325
(87) Internationale Veröffentlichungsnummer: WO9510228

(56) Entgegenhaltungen:
- WO-A-91/14947
- WO-A-93/02618
- DE-A- 3 445 047
- DE-A- 4 221 739
- US-A- 5 152 288

## Beschreibung

Die Erfindung betrifft eine Einrichtung zur Ermittlung der Wirkung gepulster primärer Magnetfelder auf einen Organismus.

Auf einen Organismus einwirkende, pulsierende Magnetfelder sind zum Beispiel von besonderem Interesse für den Ionen-Transport aus intrakorporalen Flüssigkeiten in und durch die sie umgebenden Gefäßwände und Membranen von Organismen. Vorrichtungen für diesen Zweck sind zum Beispiel in der DE 42 21 739 A1 beschrieben. Diese Offenlegungsschrift beschreibt auch Einrichtungen zur Bestimmung der Wirkung solcher Magnetfelder. Hierfür wird entweder während der Pulspausen der Organismus mit einem etwa sinusförmigen Signal im Frequenzbereich um 100 kHz beaufschlagt und das entsprechende Empfangssignal mit einer Meßspule aufgenommen oder mit der Meßspule über eine Ausblendschaltung das Sekundärfeldsignal erfaßt, das jeweils nach einem Impuls des primären Magnetfeldes durch das im Organismus entstehende sekundäre und abklingende Magnetfeld in der Meßspule induziert wird.

Die Erfindung betrifft eine Weiterbildung einer solchen Einrichtung mit dem Ziel den Aussagegehalt der Meßergebnisse zu erhöhen.

Dies wird bei einer Einrichtung zur Ermittlung der Wirkung gepulster primärer Magnetfelder auf einen Organismus, bei der für vom Organismus über einen, vorzugsweise als Meßspule für Sekundärfeldsignale ausgebildeten Meßaufnehmer abgeleitete Signale eine Auswerteschaltung vorgesehen ist, nach der Erfindung dadurch erreicht, daß die Auswerteschaltung mit einer Speichereinrichtung versehen ist, daß die Speichereinrichtung einen Speicher aufweist dem eine derart ausgebildete Steuereinrichtung zugeordnet ist, daß das Einschreiben von mehreren zeitlich aufeinanderfolgenden Einzelsignalen in den Speicher in der Weise erfolgt, daß diese im Speicher zu einem Summensignal zusammengeführt werden, und daß dieses Summensignal aus mehreren Einzelsignalen als Ausgangssignal der Auswerteschaltung vorgesehen ist.

Für die Wirkungserfassung bestehen mehrere vorteilhafte Auswertungsmöglichkeiten. So kann in der Auswerteschaltung der Amplitudenwert von Sekundärfeldsignalen und/oder der Energieinhalt von Sekundärfeldsignalen ermittelt werden. Eine vorteilhafte Ausgestaltung besteht darin, daß der Auswerteschaltung eine Schaltung zur Mittelwertbildung aus mehreren Einzelwerten zugeordnet ist. Diese ist vorzugsweise als Korrelator ausgebildet, der für die Einzelwerte eine algebraische und für störende Signale eine geometrische Addition durchführt. Auf diesem Wege läßt sich das Signal/Geräuschverhältnis erheblich anheben und damit die AussageGenauigkeit des Meßergebnisses erhöhen.

Die erfindungsgemäße Ausbildung ermöglicht es auch die Auswerteschaltung derart auszubilden, daß sie jeweils den zu Beginn und/oder zum Ende einer Beaufschlagung des Organismus auftretenden Wert dem Speicher zuführt. Nach einer weiteren vorteilhaften Ausgestaltung wird die Auswerteschaltung derart ausgebildet, daß Differenzsignale aus Sekundärfeldsignalen gebildet und abgespeichert werden.

Als Speicher wird mit Vorteil ein von der Einrichtung trennbarer Speicher, der auch ein zusätzlicher Speicher sein kann, vor allem eine Speicher-Chipkarte vorgesehen. Es ist auch möglich den Speicher in mehrere Bereiche zu unterteilen, von denen einer für die Abspeicherung ermittelter Werte, einer für die Abspeicherung von Behandlungsdaten und einer als zugriffsgeschützter Bereich für organismusbezogene, persönliche Daten vorgesehen ist.

Da die sekundären magnetischen Felder sehr schwach sind, empfiehlt sich zur Sicherstellung vergleichbar im Speicher auswertbarer Einzelsignale Vorkehrungen zu treffen. Dies ist um so besser der Fall, je besser das Signal/Geräusch-Verhältnis ist. Eine vorteilhafte Lösung hierfür besteht darin, daß beispielsweise der zur Aufnahme des Sekundärfeld-Signals aus dem Organismus dienenden Meßspule, wie an sich bekannt, eine weitere Spule, abseits von der Meßspule, derart zugeordnet wird, daß sie im wesentlichen nur von den auch die Meßspule durchsetzenden magnetischen Störfeldern durchsetzt wird, und daß beide Spulen hinsichtlich ihrer auf die Störfelder zurückgehenden Ausgangssignale elektrisch in Differenzschaltung mit dem Eingang der Auswerteschaltung verbunden sind. Beispielsweise kann die weitere Spule im gewissen Abstand über der Meßspule angeordnet werden, so daß sie im wesentlichen nur die Raumstörsignale aufnimmt, jedoch praktisch kein Sekundärfeld-Signal, während die Meßspule außer dem Raumstörsignal auch das Sekundärfeldsignal aufnimmt. Sind beide Spulen in ihren Abmessungen und Windungszahlen so aufeinander abgestimmt, daß sie hinsichtlich der magnetischen Raumstörungen zumindest etwa gleiche Ausgangssignale abgeben, so können sie elektrisch gegensinnig zusammengeschaltet und mit dem Eingang der Auswerteschaltung verbunden werden. Im Speicher ist dann ein wesentlich verbessertes Signal für die weitere Verarbeitung verfügbar. Man kann die von den beiden Spulen erhaltenen Signale auch durch vorzugsweise einstellbare Dämpfungsglieder oder Regelverstärker hinsichtlich der Raumstörung einander angleichen. Zur Erhöhung des Signal/Geräusch-Verhältnisses in der Auswerteschaltung kann auch eine magnetische Abschirmung der Meßspule gegen äußere magnetische Störfelder (magnetische Raumstörung) vorgesehen werden.

In diesem Zusammenhang sind auch Mittel zur Behebung des Einflusses von Abstandsänderungen zwischen Meßaufnehmer und Organismus während des Meßvorgangs, insbesondere durch Atmungsbewegungen vorteilhaft. Eine Möglichkeit hierfür ist eine feste Verankerung des Meßaufnehmers am Organismus. Eine andere, gegebenenfalls zusätzliche Möglichkeit besteht darin, eine Schaltung zur Ableitung eines Triggersignals aus Bewegungen des Organismus vorzusehen und die Auswerteschaltung mit einem Triggerteil zu versehen, der das Triggersignal zugeführt wird, um dadurch die Auswerteschaltung nur zu solchen, durch das Triggersignal bestimmten, Zeiten, in denen der gleiche Abstand zwischen dem Meßaufnehmer, wie der Meßspule und relevantem Organismusbereich gegeben ist, wirksam zu schalten. Die Ableitungsschaltung kann beispielsweise eine Lichtschranke sein.

In Weiterbildung der Erfindung ist, insbesondere zusätzlich, als Meßaufnehmer ein elektrochemischer Aufnehmer beziehungsweise Sensor für vom Organismus abgegebene Gase oder für organismusspezifische Flüssigkeiten vorgesehen. Bei einem Meßaufnehmer für Gase wird dieser zweckmäßig mit und in einer Atemmaske integriert. Dieser Meßaufnehmer ist mit Vorteil ein Sensor für Chlorwasserstoff und/oder Stickstoffmonoxid.

Nach einer anderen Weiterbildung der Erfindung ist, insbesondere zusätzlich, als Meßaufnehmer ein Radiationsthermometer für die Temperatur des Organismus vorgesehen ist, das in seinem Ausgang den Temperaturwert als elektrisches Signal abgibt. Es hat sich als vorteilhaft erwiesen das Radiationsthermometer als Meßorgan zur Messung der Temperatur des Organismus in einer Körperöffnung, insbesondere im Ohr des Organismus auszubilden.

Zweckmäßig wird die Einrichtung in der Weise konstruktiv ausgebildet, daß sie mit einem Gerät zur Beaufschlagung eines Organismus mit pulsierenden Magnetfeldern eine Geräteeinheit bildet.

Vorteilhaft ist es ferner, wenn in einer Einrichtung, deren Auswerteschaltung ein weiterer, insbesondere trennbarer Speicher zugeordnet ist, eine Sperreinrichtung vorgesehen ist, die nur bei angeschaltetem weiterem Speicher die Aktivierung des Geräts zuläßt.

Als vorteilhaft hat es sich erwiesen, wenn in der Einrichtung eine Sperreinrichtung vorgesehen ist, die eine Aktivierung des Geräts nur am späten Vormittag und/oder am späten Nachmittag zuläßt.

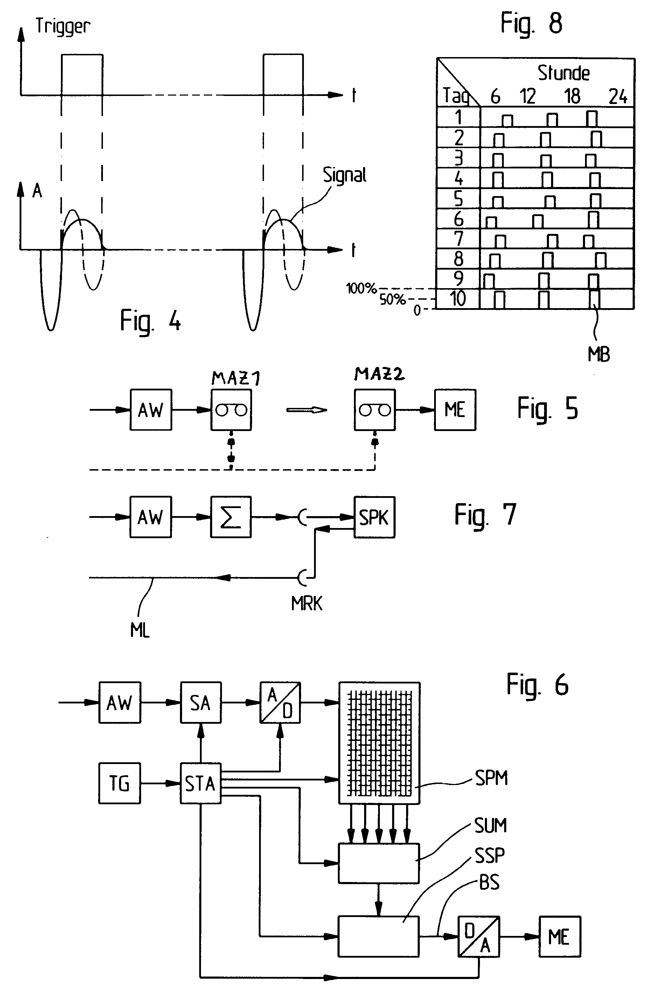

Durch die US-PS 5 152 288 ist ein Gerät und ein Verfahren zur Messung schwacher ortsabhängiger und zeitabhängiger magnetischer Felder bekannt, bei dem mittels einer Vielzahl von, dort als "superconducting quantum interference devices (SQIDS)" bezeichneten, Meßaufnehmern schwache Magnetfelder, die von dem zu untersuchenden Organismus ausgehen, erfaßt werden. SQUIDS sind unter anderem in ihrem Aufbau, ihrer Wirkungsweise und ihrer Anwendung in dem Buch "Mikroelektronische Sensoren" von Waldmann und Ahlers, VEB Verlag Technik/ Berlin, 1. Auflage 1989, Seiten 148/149 mit Literaturnachweisen beschrieben. Wie die Figur 7 obiger Patentschrift zeigt werden die mit den SQUIDS erfaßten Signale unter anderem einem Speicher zugeführt, von dem aus die abspeicherten Werte zur Ableitung eines anatomischen Bildes des Untersuchungsobjekts oder eines Modelles des Untersuchungsobjekts weiter verwendet werden. Es handelt sich demzufolge, wie weitere Ausführungen in der Patentschrift ausweisen um ein Gerät für Tomographie und nicht um ein Gerät zur Bestimmung der Wirkung gepulster primärer magnetischer Felder auf einen Organismus und es ist dort auch nicht die erfindungswesentliche Speichereinrichtung mit ihrer besonderen Steuereinrichtung gegeben.

Nachstehend wird die Erfindung näher erläutert. In der diesem Zweck dienenden Zeichnung zeigt
- Figur 1: ein Blockschaltbild eines Geräts zur Erzeugung pulsierender Magnetfelder auf einen Organismus,
- Figur 2: einen Impulsfahrplan,
- Figur 3: eine erfindungsgemäße Einrichtung,
- Figur 4: den zeitlichen Amplitudenverlauf eines gemessenen Sekundärfeldsignals,
- Figur 5: eine analog arbeitende erfindungsgemäße Einrichtung mit einem Magnetbandspeicher,
- Figur 6: eine digital arbeitende erfindungsgemäße Einrichtung mit einem matrixartig aufgebauten Speicher,
- Figur 7: eine erfindungsgemäße Einrichtung mit einer externen Speicherkarte,
- Figur 8: eine vorteilhafte Ausgestaltung einer externen Speicherkarte für die bei der Behandlung eines Organismus eintretenden Änderungen,
- Figur 9: ein Blockschaltbild für eine erfindungsgemäße Einrichtung mit einer Zeitsperre,
- Figur 10: einen Schnitt durch einen bekannten, als Meßaufnehmer in einer erfindungsgemäßen Einrichtung einsetzbaren, Gassensor und
- Figur 11: eine Atemmaske mit einem Gassensor.

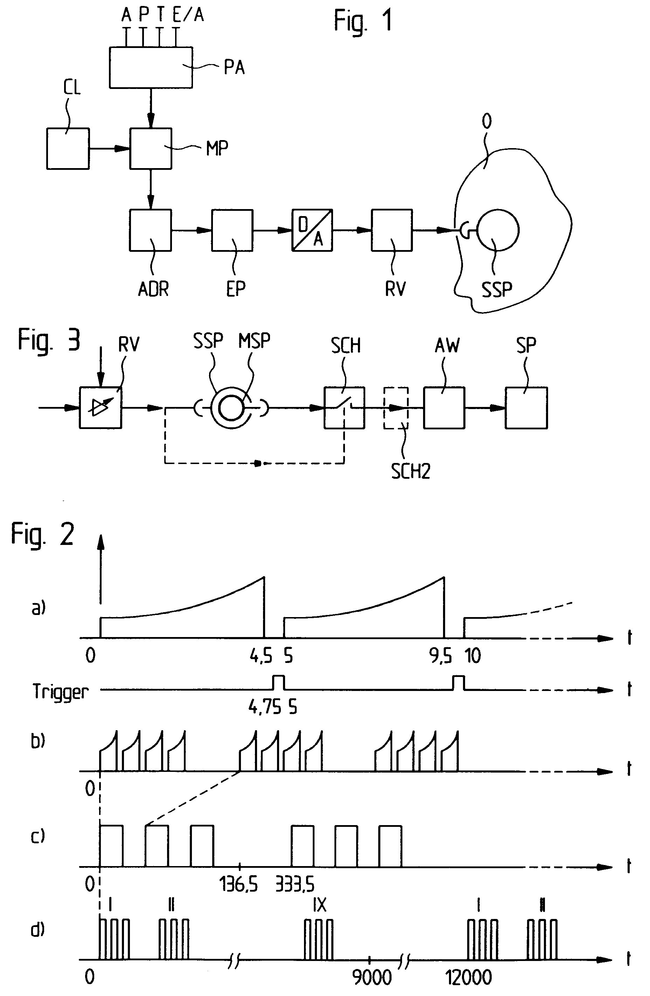

Das Blockschaltbild nach Figur 1 entspricht dem in der einleitend erwähnten DE 42 21 739 A1 dargestellten und beschriebenen. Danach wird eine Sendespule SSP mit entsprechenden Strömen gespeist. Die Sendespule erzeugt in dem Organismus O ein gleichartiges (primäres) Magnetfeld, das gegenüber dem Strom in der Sendespule phasenverschoben ist. Dieses primäre Magnetfeld erzeugt im Organismus O eine (sekundäre) Spannung, die ihrerseits einen entsprechenden (sekundären) Stromfluß im Organismus auslöst, deren zeitlicher Verlauf die 1. Ableitung des primären Magnetfeldverlaufs ist. Durch den (sekundären) Strom baut sich ein (sekundäres) Magnetfeld auf, dessen zeitlicher Verlauf dem sekundären Stromflußverlauf entspricht. Das sekundäre Magnetfeld induziert in einer Meßspule MSP (Figur 3) einen Strom, der die 1. Ableitung des zeitlichen Verlaufs des sekundären Stromes und damit die 2. Ableitung des primären Stromes ist, der in der Sendespule geflossen ist.

Die Figur 2 zeigt in einem Impulsfahrplan den zeitlichen Verlauf des das primäre Magnetfeld bewirkenden Stromes in der Sendespule SSP. Im Impulsfahrplan sind zum besseren Verständnis erprobte Werte für die Dauer der einzelnen Impulse und Impulspausen in Millisekunden eingetragen.

Im Impuls-Fahrplan ist unter a) die Form des sogenannten Grundpulses wiedergegeben, der eine Wiederholungsfrequenz von z.B. 200 Hz aufweist und einen exponentiellen Dachverlauf hat. Der Grundpuls wird aber nicht ständig ausgesandt. Vielmehr folgt, wie unter b) gezeigt, auf jeweils vier Grundpulse eine Impulspause. Drei solche Impulspakete, bestehend aus jeweils vier Grundpulsen und den entsprechenden Impulspausen werden, wie unter c) gezeigt, nacheinander ausgesandt, gefolgt von einer weiteren Impulspause. Wie unter d) dargestellt, werden neun solche Dreierpakete nacheinander ausgesandt, gefolgt von einer weiteren Impulspause. Dann wiederholt sich der Ablauf für eine Dauer von mehreren Minuten, beispielsweise von zwei Minuten.

Die von dem Primärsignal und dessen Magnetfeld im Organismus O ausgelösten Sekundärfelder werden mit einer erfindungsgemäßen Einrichtung erfaßt, für die in der Figur 3 ein Blockschaltbild gezeigt ist. Der Grundaufbau entspricht beim Beispiel dem von Figur 14 der einleitend genannten DE 42 21 739 A1. An den Ausgang der Ausblendschaltung SCH ist jedoch eine Speicherschaltung SP angeschlossen, die der Speicherung von Sekundärfeld-Signalen dient, die nach den einzelnen Grundpulsen über die Meßspule MSP empfangen werden. Es ist das jeweils in der Impulspause von ca. 0,5 Millisekunden nach dem einzelnen Grundpuls (vergl. Figur 2a). Da bei der Rückführung des Grundpulses auf den Wert Null eine Stromspitze in der Meßspule auftritt, die nicht dem gesuchten Sekundärfeld-Signal entspricht, wird erst nach dem Abklingen dieser Stromspitze die Verbindung zum Speicher SP hergestellt. Dies ist z.B. dadurch erreichbar, daß aus dem Grundpuls, so wie in der Figur 2 eingezeichnet, ein entsprechend zeitverzögerter Triggerimpuls abgeleitet wird, der für die Durchschaltung von der Meßspule zur Auswerteschaltung AW mit dem Speicher SP dient, und zwar für die Zeit bis zum Beginn des nächsten Grundpulses. Auf diese Weise fällt eine Folge von Sekundärfeld-Signalen an, die in dem Speicher SP für die weitere Auswertung abgelegt sind.

Der Verlauf eines solchen Sekundärfeld-Signals ist in der Figur 4 gezeigt, in der auf der Abszisse Zeitwerte und der Ordinate Amplitudenwerte aufgetragen sind. Man sieht daraus, daß das Sekundärfeld-Signal relativ schwach ist und demzufolge störende Signale, wie Rauschen und äußere Störfelder zu nicht unerheblichen Meßfehlern führen können. Das ist gleichbedeutend einem unzureichenden Signal/Geräusch-Verhältnis. Die in der Figur 4 gezeigte Form ist die eines gemessenen Sekundärfeld-Signals. An sich kann man die Sekundärfeld-Signale aus beliebigen Impulsen der primären Impulsfolge ableiten. Es hat sich aber als vorteilhaft erwiesen für die Ableitung die Folge von Grundpulsen zu verwenden, wodurch sich für die in der Figur 2 dargestellten Verhältnisse eine Folgefrequenz von z.B. 200 Hz ergibt.

Durch den Triggerimpuls wird der Ausblendschalter SCH nur während der Zeit des hier interessierenden Sekundärfeld-Signals geschlossen. Dadurch werden nicht nur die gestrichelt eingezeichneten Spannungsspitzen am Ende des einzelnen Impulses - vergl. Figur 4 - unterdrückt, sondern auch sonstige in dem Unterdrückungszeitraum anfallende unerwünschte Signale.

Bei einer Messung über einen gewissen Zeitraum fallen zeitlich nacheinander Sekundärfeld-Signale an, und zwar in der Regel als Signale in Analogform. Wird dann als Speicher, so wie in der Figur 5 gezeigt, z.B. ein Magnetband-Aufzeichnungsgerät verwendet, so werden die einzelnen Signale auf dem Magnetband geometrisch nacheinander aufgezeichnet. Sie können demzufolge in einfacher Weise weiterverarbeitet werden. Vor allem kann eine Folge von einzelnen Aufzeichnungen mittels eines Korrelationsverfahrens zu einem Summensignal zusammengefaßt werden, das ein erheblich besseres Signal/Geräusch-Verhältnis aufweist, als das einzelne aufgezeichnete Signal. Die Korrelationstechnik ist an sich aus der Nachrichten-Übertragungstechnik allgemein bekannt und zum Beispiel in dem Buch "Korrelationstechnik" von Lange, 1960, VEB-Verlag, Berlin, vor allem auf den Seiten 348 und 353 hinsichtlich von Details ausführlich und mit Quellennachweisen behandelt. Wesentlich im vorliegenden Anwendungsfall ist, daß aufeinanderfolgende Sekundärfeldsignale für eine gewisse Anzahl aufeinanderfolgender Grundpulse im Amplitudenverlauf fast identisch sind und damit einer algebraischen Addition zugänglich sind, z.B. dadurch, daß man die aufeinanderfolgenden Sekundärfeld-Signale nacheinander ausliest und die Ausleseergebnisse in einem gemeinsamen Speicher im Sinne einer reinen Addition phasensynchron übereinander schreibt. Störsignale wie Rauschen und Kurzeitstörungen sind dann zwangsläufig nicht phasensynchron und addieren sich weitgehend geometrisch. Phasensynchrone Störsignale lassen sich bei dem Verfahren im übrigen selektiv aus dem Summensignal entfernen, indem man diese mit einer Zeitfenstertechnik aus dem Summensignal sozusagen herausschneidet. Man erhält damit ein für die weitere Auswertung gut brauchbares Signal. In der Figur 5 ist eine solche Summation in einen gemeinsamen Speicher schematisch mit dargestellt. Vom Ausgang des Magnetband-Aufzeichnungsgeräts MAZ1 wird ein weiteres Magnetband-Aufzeichnungsgerät MAZ2 gespeist. Das als geschlossene Schleife ausgebildete Magnetband von MAZ2 hat eine solche Schleifenlänge und ist in seiner Bandgeschwindigkeit so gegenüber MAZ1 abgestimmt, daß aufeinanderfolgende Sekundärfeldsignale von MAZ1 stets auf den gleichen Bandabschnitt in MAZ2 geschrieben werden. Dabei ist darauf zu achten, daß das Band in MAZ2 zwischen den einzelnen Einschreibungen nicht gelöscht wird. Zur Sicherstellung eines exakten Zeit- bzw. Phasen-Synchronismus empfiehlt sich - wie durch eine gestrichelt eingezeichnete Leitung angedeutet - die Synchronisierung z.B. mit dem Triggersignal, das zur Betätigung der Ausblendschaltung SCH (Figur 3) dient.

Das Summensignal kann über einen Auslesekopf von MAZ2 zur eigentlichen Meßeinrichtung ME gebracht werden. Die Meßeinrichtung ME kann ein Oszilloskop, ein Meßgerät zur Bestimmung der Maximalamplitude des Signals oder ein Integrator zur Bestimmung des - vergl. Figur 4 - von dem Sekundärfeldsignal überdeckten Flächenbereichs, und damit von dessen Energieinhalt sein.

Der Phasen- und Zeit-Synchronismus zwischen MAZ1 und MAZ2 kann auch dadurch sichergestellt werden, daß die Synchronisierung von MAZ2 aus dem Signalausgang von MAZ1 abgeleitet wird. Dafür kann zum Beispiel in MAZ1 zu dem Sekundarfeldsignal noch das Triggersignal oder ein Synchronsignal aufgezeichnet werden, das in MAZ2 zur Synchronisation ausgewertet wird. Dieser Weg ermöglichtes im übrigen, die eigentliche Signalaufnahme über die Meßspule MSP von der eigentlichen Auswertung über MAZ2 zeitlich zu trennen.

Anstelle einer Analogspeicherung ist auch eine Speicherung auf digitaler Basis mit Vorteil anwendbar. In diesem Fall werden, wie in der Figur 6 gezeigt, die Sekundärfeld-Signale in einem Sampler SA mit entsprechend hoher Abtastfrequenz abgetastet. Die einzelnen Abtastproben werden in einem sogenannten Analog-Digital-Wandler A/D in ein Digitalsignal umgewandelt. Man erhält damit in an sich bekannter Weise eine Folge von Digitalsignalen, deren jedes den Amplitudenwert einer Abtastprobe darstellt und die in einem Speicher sozusagen für die weitere Verarbeitung abgelegt werden können. Dabei empfiehlt es sich die Digitalsignale aufeinanderfolgender Sekundärfeldsignale in aufeinanderfolgende Zeilen eines nach Art einer Matrix organisierten Speichers SPM zuschreiben, so daß die einzelnen digital geschriebenen Sekundärfeldsignale in der Matrix übereinanderstehen. Zur Auswertung ist es dann lediglich erforderlich die in den Matrixspalten übereinander stehenden Werte in einem Addierer SUM digital zu summieren und in einem Summenspeicher SSP spaltengerecht abzulegen. Dem Summenspeicher SSP ist dann das bereinigte Signal BS entnehmbar. Dieses kann über einen Digital-Analog-Wandler in an sich bekannter Weise in ein Analogsignal umgeformt und so, wie anhand der Figur 5 erläutert, verwendet werden. Man kann es jedoch auch in an sich bekannter Weise in der digitalen Signalebene entsprechend auswerten.

Das Auslesen aus der Speichermatrix kann seriell erfolgen, ebenso wie das Einschreiben der Ergebnisse aus SUM in der Summenspeicher SSP. Man kommt dadurch in an sich bekannter Weise mit weniger Verbindungen zwischen SPM, SUM und SSP aus.

Die für den Ablauf in an sich bekannter Weise erforderlichen Taktsignale für die einzelnen Schaltungsbausteine (SA,A/D,SPM,SSP,SUM,D/A) werden von einem gemeinsamen Taktgenerator TG über Steuersignal-Ableitschaltung STA in ebenfalls an sich bekannter Weise geliefert.

Die Einfügung eines Speichers in die Auswerteschaltung eröffnet auf einfache Weise eine Reihe von Auswertemöglichkeiten unterschiedlichen qualitativen und quantitativen Aussagegehalts. Die Amplitude des in der Meßspule induzierten Stromes nach jedem Impuls ist ebenso wie die Energie, die nach jedem Impuls in die Meßspule vom Organismus übertragen wird, eine wesentliche Große für die Bestimmung der Wirkung, die der einzelne Impuls auf den beaufschlagten Organismus hinsichtlich eines Ionentransports ausübt. Das gilt insbesondere für die Grundstromimpulse mit einer Wiederholungsfrequenz zwischen 100Hz und 1000 Hz, vorzugsweise 200 Hz. Die Sekundärfeld-Signale bzw. Feedbacksignale sind die 2. Ableitung der primären Signals und sind durch den beaufschlagten Organismus in ihrem Amplitudenverlauf bestimmt. Aus der 2. Ableitung lassen sich u.a. folgende Aussagen ableiten.
1. Sie ist ein Maß für eine erhöhte Elektrolythmenge (Blut, Lymphe, extrazelluläre Flüssigkeit) durch eine höhere Ionenfluß-Rekrutierung (höherer Verschiebungsstrom) im induzierten elektromotorischen Feld. Maß ist vor allem die Höhe der ersten Amplitude innerhalb des eines Zyklus von vier Impulsen der Serie 200 Imp/sec (siehe Figur 2). Das entspricht in einem Organismus physiologisch einer erhöhten zentralen und peripheren Durchblutung (erhöhte Blutgefäßdilatation und erhöhte Blutperfusion).
2. Sie ist ein Maß für die Polarisationsamplitude der betroffenene Grenzflächen durch:
   a. die Neutralisierung und evtl. Umladung des Kontaktpotentials zwischen den Phasen "flüssig" und "fest", also zwischen Elektrolyth und Gefäßwand bzw. Membran;
   b. die Größe der kapazitiven Aufladung; wobei die Menge der herangebrachten Ionen für die Grenzflächenpolarisation sowohl proportional der Elektrolythmenge (siehe 1.) als auch proportional der induzierten elektromotorischen Kraft, also auch der Größe der induzierten Fläche ist.
   Maß ist hierbei die Formveränderung der Feedback-Impulsspitzen und die abfallende Höhe der 2.,3. und 4. Impulsamplituden innerhalb des 4-Impulszyklus der Serie 200 Imp/sec. Das entspricht physiologisch der pH-Veränderung durch schnelle Protonenwanderung im elektromotorischen Kraftfeld und der Ca⁺⁺-Freisetzung aus Proteinen bei pH-Erniedrigung bzw. Ca⁺⁺-Bindungen bei pH-Erhöhung.

Die unter 1. und 2. beschriebenen Effekte sind wiederum korreliert mit:
- erhöhtem Sauerstoffpartialdruck (pO₂) der Gewebezellen,
- erhöhter Substratversorgung der Gewebezellen,
- erhöhter Metaboliten-Entsorgung der Gewebezellen,
- Forcierung der Immunsystem-Aktivität, insbesondere der Makrophagen-Tätigkeit,
- erhöhter Aktivität bestimmter Enzyme,
- erhöhter Zellregeneration, und
- erhöhtem Informationsfluß.

Die Bewertung dieser korrelierten Größen geschieht über die Messung des Flächeninhalts im Sekundärfeld-Verlauf, d.h. der Feedbackimpuls-Spannungs-Zeit-Kurve, also der Feedbackimpulsfläche aufaddiert innerhalb mehrerer Impulskaskaden, bestehend aus je 4-Impulszyklen. Dieser Flächenwert kann laufend angezeigt werden als allgemeine Bewertung der aktuellen Durchblutung. Die Änderung, insbesondere der Zuwachs, des jeweils aufaddierten Flächenwertes je Impulskaskade verglichen mit dem Flächenwert der letzten Impulskaskade ist eine entscheidende Wirkungsinformation des physikalischen Therapie-Systems oder ausgelöst durch Pharmaka (Durchblutungsförderung) und Sport und dergleichen. Es empfiehlt sich deshalb diese in einer gesonderten Anzeigevorrichtung sichtbar zu machen. Der Flächenwert nimmt pro Zeiteinheit insbesondere dann einen höheren Wert an, wenn gegenüber dem Ausgangszustand eine höhere Durchblutung und/oder bei nicht übermäßiger Polarisation der Grenzflächen ein höherer Lymphfluß stattfindet. Damit sind applizierte Amplitude und Dauer des Magnetfeldes des Medikamentes, der Bewegung u.a., also die Dosis, exakt durch die Wirkung am Organismus bewertbar.

Die Messung wird bei atmenden Organismen unter Umständen stark durch die Atmungsbewegung moduliert, weil sich dabei der Abstand zwischen Meßspule und Organismus ändert, wenn nicht hiergegen Vorkehrungen getroffen werden. Vor allem bei längeren Meßzyklen kann sich das störend bemerkbar machen. Ein Weg zur Behebung dieser Schwierigkeit besteht darin, daß die Meßspule mit Mitteln zur festen Verankerung an dem Organismus versehen wird. Eine andere Möglichkeit beruht darauf, daß die Atemfrequenz wesentlich verschieden von der Wiederholungsfrequenz der Sekundärfeldsignale ist. Man kann nun zum Beispiel mittels Infrarot-Lichtschranken bestimmte Atmungs-Zeitphasen festlegen in denen der Abstand zwischen Meßspule MSP und atmendem Organismus O innerhalb bestimmter Grenzwerte liegt und nur in diesen Zeitphasen die von MSP aufgenommenen Sekundärfeld-Signale zur Auswertung zuführen, beispielsweise indem SCH ein weiterer Ausblendschalter SCH2 zugeordnet wird - in der Figur 3 gestrichelt eingezeichnet - der nur in diesen Zeitphasen durchschaltet.

Die erfindungsgemäße Ausgestaltung eröffnet auch die Möglichkeit die Langzeitwirkung einer Magnetfeldbehandlung festzustellen und festzuhalten. Diesem Zweck dient ein bezogen auf das Gerät - externer Speicher, wie eine Patientenkarte. Diese Patientenkarte kann eine Karte mit einem Speicherchips ein, wie sie an sich allgemein bekannt ist, beispielsweise durch das Buch "Chipkarten -Technik, Sicherheit, Anwendungen" von Fietta, Verlag Dr. AlfredHüthig, 1989, Heidelberg (insbesondere der Hinweis auf Seite 132 für solche Zwecke). Zweckmäßig ist es, wenn das Gerät zum Einspeichern von Daten in den externen Speicher, wie eine Patientenkarte mit einer Sperrvorrichtung versehen wird, die nur dann die Inbetriebnahme des Geräts ermöglicht, wenn der externe Speicher sich in der für ihn vorgesehenen Aufnahme des Geräts befindet und schreibaufnahmefähig ist. In der Figur 7 ist dies durch einen Rückmeldekontakt MRK beziehungsweise Schalter angedeutet, der über eine Meldeleitung ML das Gerät zum Betrieb freigibt. Solche Sperren sind zum Beispiel aus der Technik der Floppy-Disk-Laufwerke von Personal-Computern bekannt, so daß sich ein weiteres Eingehen auf Details erübrigt.

Auf einer solchen Patientenkarte wird - wie als Beispiel in Figur 8 dargestellt - die Summe aller Differenzen der Impulszyklen, also aller Zuwächse, über eine Therapiezeit bzw. Meßzeit von zum Beispiel 7 Minuten pro Behandlung aufgezeichnet. Das kann zum Beispiel mit einer Zahl zwischen 1 bis 100 erfolgen, die den prozentualen Wachstum gegenüber dem Erstzyklus (= Erstmessung absolut oder relativ pro Tag) darstellt (100% Wachstum heißt also doppelt so große Feedback-Amplitude, entsprechend einem ca. doppelt so großen Blut- oder Lymphvolumen am Schluß der Behandlung verglichen mit der Erstmessung. Dies kann auch im Sinne einer umschaltbaren Darstellung für den Tageserfolg gewertet werden.

Wird die Karte, wie in der Figur 8 vorausgesetzt, als visuell lesbare Karte ausgeführt, empfiehlt es sich die Karte hinsichtlich der Daten so aufzubauen, daß Daten eines Behandlungstages jeweils in einer Zeile festgehalten werden und diese Tageszeilen untereinander geschrieben werden. Für drei Messungen pro Tag beziehungsweise 24 Stunden und zehn aufeinanderfolgende Behandlungstage ergibt sich dann die gezeigte Darstellungsform. Die Differenzen aus dem jeweiligen Anfangswert und dem zugehörigen Endwert sind dabei beispielsweise als Meßbalken MB eingetragen.

Für die Messung hat es sich als vorteilhaft erwiesen, wenn etwa einhundert aufeinanderfolgende Sekundärfeld-Signale jeweils zu einem Meßwert zusammengefaßt werden. Das entspricht einer Meßdauer von etwa 500 Millisekunden. Wird diese Messung jeweils zu Anfang und zum Ende einer z.B. sieben Minuten anhaltenden Behandlung eines Organismus durchgeführt, so kann man durch Differenzbildung zwischen den beiden Meßwerten, die durch die jeweilige Behandlung im Organismus eingetretene Veränderung bestimmen. Die Korrelationsauswertung von jeweils einhundert Sekundärfeld-Einzelsignalen ergibt eine erhebliche Verbesserung des Signal/Geräusch-Verhältnisses.

Die Verwendung eines Speichers bringt noch eine weitere Auswertemöglichkeit für das Sekundärfeld-Signal. Wird an den Speicher SP (Figur 3) ein Differenzierglied angeschaltet, so ist an dessen Ausgang ein Signal verfügbar, das über die Feinstruktur des Sekundärfeld-Signals Aussagen ermöglicht, wie es in ähnlicher Form für die Auswertung von Elektro-Kardiogrammen seit Jahren bekannt ist. So lassen sich nicht nur die Einwirkungsmechanismen besser erkennbar machen, sondern auch die Wirkungen von mittels der Magnetfelder in einem Organismus zu transportierenden Ionen. Das Differenzierglied wird zweckmäßig dem Speicher SP nach- und der eigentlichen Meßeinrichtung vorgeschaltet. Sein Ausgangssignal kann auch in einem Speicher zur weiteren Verarbeitung abgelegt werden. In der Figur 4 ist der Verlauf eines durch Differentation erhältlichen Signals punktiert eingezeichneten. Es ist auch möglich die Differentation vor den Speicher einzuschalten und das Differentations-Ergebnis in den Speicher für die weitere Verwertung einzuspeichern.

Wie der Erfindung zugrundeliegende Untersuchungen zeigten, ist bei der Beaufschlagung eines Organismus der Zeitpunkt von nicht unwesentlicher Bedeutung. Da durch Magnetfelder das Schlafhormon Melatonin unterdrückt werden kann, ist bei Schlafbedürfnis eine Beaufschlagung zu unterlassen bzw. bei einem Wachhaltebedürfnis angezeigt. Da weiterhin der Adrenalinspiegel für Wirkung von Einfluß ist, empfiehlt es sich den Zeitpunkt auf den späten Vormittag (11⁰⁰ +/- 2 Stunden) und/oder auf den späten Nachmittag (16⁰⁰ +/- 2 Stunden) zu legen, da in diesen Zeiträumen der Adrenalinspiegel im circadianen Rhythmus des Menschen am höchsten ist.

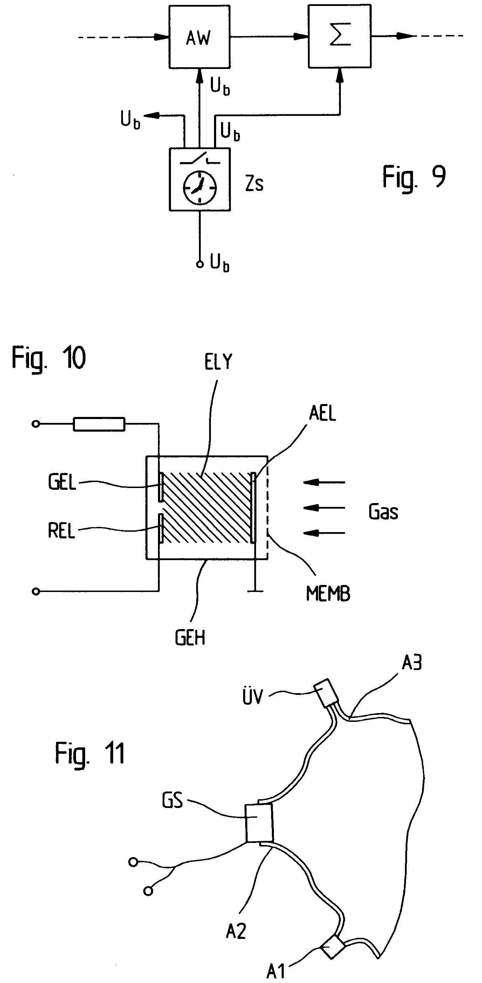

Das kann dadurch realisiert werden, daß so wie in der Figur 9 schematisch gezeigt ein Zeitschalter ZS vorgesehen wird, der beispielsweise die Betriebsspannungs U_{b} für die Auswerteschaltung AW und/oder weitere Schaltungsteile der Einrichtung wie den Summierspeicher, insbesondere aber den Generator für die Speisung der Magnetfeldspule außerhalb der erwähnten Zeiten unterbricht und somit das Gerät außer Betrieb hält.

Wie der Erfindung zugrundeliegende Untersuchungen weiterhin zeigten, führt die Magnetfeldexposition eines Organismus aufgrund eines erhöhten Sauerstoffpartialdrucks im Gewebe zu einer erhöhten Sauerstoffdiffusion. Sie führt nämlich zu einer Dilatation der Blutgefäße. Ebenso tritt eine Temperaturerhöhung ein. Die Einzelsignale können deshalb außer mit einem auf das sekundäre Magnetfeld ansprechenden Meßaufnehmer auch mit Chemosensoren, insbesondere Gassensoren und/oder auf Wärmestrahlung ansprechende Sensoren abgeleitet werden verwendbar. Diese unterschiedlichen Sensoren können für sich allein, jedoch auch in Kombination miteinander angewendet werden.

Als besonders wirkungsvoll hat sich die Einzelsignalableitung aus der Atemluft mit Gassensoren für die Gase Stickstoffmonoxid, Chlorwasserstoff und Kohlenmonoxid erwiesen. Solche Sensoren sind an sich und in ihrer Schaltungstechnik allgemein bekannt, z.B. durch das vorstehend erwähnte Buch "Mikroelektronische Sensoren" Seiten 111 bis 137.

Die Figur 10 zeigt eine schematischen Schnitt durch einen Gassensor wie er beispielsweise in dem Gerät G 818 der "Gesellschaft für Gerätebau" in Dortmund. In einem eine gasdurchlässige Membran MEMB aufweisenden Gehäuse GEH sind eine Arbeitselektrode AEL, eine Referenzelektrode REL und eine Gegenelektrode GEL angeordnet. Der Raum zwischen den Elektroden ist durch einen entsprechenden Elektrolyten ELY ausgefüllt, dessen Reaktion mit dem durch die Membran in den Sensor eingeführten Gas eine Änderung der zwischen den Elektroden gegebenen elektrischen Werte auslöst. Das Gerät G 818 ist u.a. für die Stickstoffmonoxid-Messung bei Kraftfahrzeugmotoren bekannt. Dieser Sensor ist ein Halbleitersensor bei dem der ihn durchfließende Strom als Meßgröße dient. Der erzeugt an einem von ihm durchflossenen Widerstand eine der Gaskonzentration entsprechende Spannung, die als Signal dient.

Zweckmäßig wird der Gassensor in eine Atemmaske integriert, die bei einem erprobten, für die Umschließung des Nasen- und Kinnbereichs eines Menschen bestimmten Muster, wie in der Figur 11 gezeigt, drei Anschlüsse aufwies. Ein erster Anschluß A1 dient der Verbindung mit einer nicht näher dargestellten Druckpumpe, die Umgebungsluft in die Atemmaske drückt. Ein zweiter Anschluß A2 enthält den Gassensor GS, von dem eine elektrische Leitung für die Abnahme der elektrischen Größe des Gassensors zum nicht dargestellten Gerät führt. Ein dritter Anschluß A3 enthält ein Überdruckventil ÜV, das gleichbleibenden Druck in der Atemmaske und dem Gassensor damit eindeutig reproduzierbare Meßverhältnisse sicherstellt. Der durch die an die Atemmaske angeschlossene, in der Zeichnung nicht dargestellte, Druckpumpe erzeugte Druck sollte gering sein und nur so hoch, daß ein sicherer Austritt der Atemluft im Bereich des Gassensors GS gewährleistet ist. Als vorteilhaft hat sich ein Überdruck gegenüber der Außenluft zwischen etwa 20 und etwa 80 Millibar erwiesen.

Anstelle der dargestellten Atemmaske mit Druckpumpenanschluß ist auch eine ohne Druckpumpe anwendbar, die vom Type der Gasmaske ist und ein Atemeinlaßventil und ein Atemluftauslaßventil hat. Der Gassensor ist im Luftweg des Atemauslasses, ähnlich zur Figur 11 anzuordnen. Dieser Atemmaskentyp erfordert aber einen gewissen zusätzlichen Energieaufwand des Organismus beim Atmen.

Die durch eine Magnetfeldbeaufschlagung ausgelöste Änderung der Atemluft erfolgt etwas zeitverzögert gegenüber dem Einwirkungszeitraum (einige Minuten) weil nicht nur der Organismus, sondern auch der Gassensor eine gewisse Zeit für das Ansprechen benötigt. Die Reaktion ist aber deutlich erkennbar, vor allem dann, wenn nach der anhand der Figur 3 erläuterten Zeitfenstertechnik nur Nutzsignale für die Messung ausgeblendet und als Einzelsignale dem Speicher zur Summation zugeführt werden. Man kann bei Visualisierung des Signals am Ausgang mittels oszillographischer Wiedergabe deutlich ein die Gaskonzentrationsänderung wiedergebendes Signal erkennen, dessen Dauer relativ gut der vorausgehenden Einwirkungszeit des Magnetpulses entspricht.

Anstelle der Auswertung von Änderungen in vom Organismus abgegebenen Gasen kann auch eine Auswertung von Änderungen von Körperflüssigkeiten, insbesondere von darin enthaltenen Enzymen vorgesehen werden. Das erwähnte Buch "Mikroelektronische Sensoren" beschreibt und erläutert auch solche Sensoren, so daß eine diesbezügliche Detailbeschreibung wegen des Bekanntseins entfallen kann.

Der erfindungsgemäßen Einrichtung kommt vor allem auch deshalb ein besondere Bedeutung zu, weil sie eine Überwachung und Kontrolle der Magnetfeldexposition ermöglicht. Überlange Exposition kann nämlich die Regelkapazität eines Organismus (z.B. Ca⁺⁺-Überschuß in Zellen) übersteigen. In diesem Fall sind pathologische Auswirkungen zu erwarten. Allerdings kann dann die Diffusion von Stickstoffmonoxid in der Lunge eines Organismus als Indikator für unphysiologische Momente dienen.

## Patentansprüche

1. Einrichtung zur Ermittlung der Wirkung gepulster primärer Magnetfelder auf einen Organismus, bei der für vom Organismus über einen, vorzugsweise als Meßspule für Sekundärfeldsignale ausgebildeten Meßaufnehmer abgeleitete Signale eine Auswerteschaltung vorgesehen ist, dadurch gekennzeichnet, daß die Auswerteschaltung mit einer Speichereinrichtung versehen ist, daß die Speichereinrichtung einen Speicher aufweist dem eine derart ausgebildete Steuereinrichtung zugeordnet ist, daß das Einschreiben von mehreren zeitlich aufeinanderfolgenden Einzelsignalen in den Speicher in der Weise erfolgt, daß diese im Speicher zu einem Summensignal zusammengeführt werden, und daß dieses Summensignal aus mehreren Einzelsignalen als Ausgangssignal der Auswerteschaltung vorgesehen ist.

2. Einrichtung nach Anspruch 1, dadurch gekennzeichnet, daß in der Auswerteschaltung eine Ausblendschaltung zwischen dem Meßaufnehmer für die Einzelsignale und dem Speicher vorgesehen ist, und daß für die Ausblendschaltung eine Steuerung vorgesehen ist, die ein Einschreiben von Einzelsignalen nur in Ruhezeiten nach einem primären Magnetfeldpuls ermöglicht.

3. Einrichtung nach Anspruch 1 oder 2, dadurch gekennzeichnet, daß die Steuerung des Speichers derart ausgebildet ist, daß das Summensignal der Mittelwert aus mehreren Einzelwerten ist.

4. Einrichtung nach Anspruch 3, dadurch gekennzeichnet, daß die Schaltung zur Mittelwertbildung als Korrelator ausgebildet ist, der für die Einzelwerte eine algebraische und für störende Signale eine geometrische Addition durchführt..

5. Einrichtung nach einem der Ansprüche 1 bis 4, dadurch gekennzeichnet, daß die Auswerteschaltung, der Speicher und die Steuerung derart ausgebildet sind, daß der Amplitudenwert von Einzelsignalen ermittelt wird.

6. Einrichtung nach einem der Ansprüche 1 bis 4, dadurch gekennzeichnet, daß die Auswerteschaltung, der Speicher und die Steuerung derart ausgebildet sind, daß der Energieinhalt von Einzelsignalen ermittelt wird.

7. Einrichtung nach einem der Ansprüche 1 bis 6, dadurch gekennzeichnet, daß die Auswerteschaltung, der Speicher und die Steuerung derart ausgebildet sind, daß Differenzsignale aus Einzelsignalen gebildet und abgespeichert werden.

8. Einrichtung nach einem der Ansprüche 1 bis 7, dadurch gekennzeichnet, daß die Auswerteschaltung und die Steuerung des Speichers derart ausgebildet sind, daß sie jeweils den zu Beginn und/oder zum Ende einer Beaufschlagung des Organismus mit pulsierenden Magnetfeldern auftretenden Summen-Signale, insbesondere in Form von Differenzsignale einem weiteren Speicher zuführt.

9. Einrichtung nach Anspruch 8, dadurch gekennzeichnet, daß der weitere Speicher ein von der Einrichtung trennbarer Speicher, insbesondere in Form einer Speicher-Chipkarte ist.

10. Einrichtung nach einem der Ansprüche 1 bis 9, dadurch gekennzeichnet, daß der Speicher in an sich bekannter Weise in mehrere Bereiche unterteilt ist, von denen einer für die Abspeicherung von ermittelten Werten, einer für die Abspeicherung von Behandlungsdaten und einer als zugriffsgeschützter Bereich für organismusbezogene, persönliche Daten vorgesehen ist.

11. Einrichtung nach einem der Ansprüche 1 bis 10, dadurch gekennzeichnet, daß zur Sicherstellung vergleichbarer Einzelsignale eine Schaltung zur Ableitung eines Triggersignals von den Bewegungen des Organismus vorgesehen ist, und daß die Auswerteschaltung mit einem Triggerteil versehen ist, der das Triggersignal zugeführt wird und das die Auswerteschaltung nur zu durch das Triggersignal bestimmten Zeiten wirksam schaltet.

12. Einrichtung nach einem der Ansprüche 1 bis 11, dadurch gekennzeichnet, daß bei einem als Meßspule ausgebildeten Meßaufnehmer und Messung von Signalen des magnetischen Sekundärfeldes, zur Sicherstellung vergleichbarer Einzelsignale im Speicher, für die Meßspule eine feste Verankerung am Organismus vorgesehen ist.

13. Einrichtung nach einem der Ansprüche 1 bis 12, dadurch gekennzeichnet, daß bei einem als Meßspule ausgebildeten Meßaufnehmer und Messung von Signalen des magnetischen Sekundärfeldes, zur Erhöhung des Signal/Geräusch-Verhältnisses in der Auswerteschaltung der zur Aufnahme des Sekundärfeld-Signals aus dem Organismus dienenden Meßspule eine weitere Spule, abseits von der Meßspule, derart zugeordnet ist, daß sie im wesentlichen nur von den auch die Meßspule durchsetzenden magnetischen Störfeldern durchsetzt wird, und daß beide Spulen hinsichtlich ihrer auf die Störfelder zurückgehenden Ausgangssignale elektrisch in Differenzschaltung mit dem Eingang der Auswerteschaltung verbunden sind.

14. Einrichtung nach einem der Ansprüche 1 bis 13, dadurch gekennzeichnet, daß bei einem als Meßspule ausgebildeten Meßaufnehmer und Messung von Signalen des magnetischen Sekundärfeldes, zur Erhöhung des Signal/Geräusch-Verhältnisses in der Auswerteschaltung die Meßspule gegen äußere magnetische Störfelder abgeschirmt ist.

15. Einrichtung nach einem der Ansprüche 1 bis 14, dadurch gekennzeichnet, daß insbesondere zusätzlich als Meßaufnehmer ein elektrochemischer Aufnehmer für vom Organismus abgegebene Gase oder für organismusspezifische Flüssigkeiten vorgesehen ist.

16. Einrichtung nach Anspruch 15, dadurch gekennzeichnet, daß bei einem Meßaufnehmer für Gase, dieser in einer Atemmaske integriert ist.

17. Einrichtung nach Anspruch 16, dadurch gekennzeichnet, daß die Atemmaske mit drei Anschlüssen versehen ist, von denen einer der Luftzufuhr mit Überdruck, einer der Aufnahme des Gassensors und einer für einen Überregler vorgesehen ist, der einen weitgehend konstanten Überdruck für den Gasdurchtritt vom Gaseintritt in den Gassensor in den Raum außerhalb der Atemmaske sicherstellt.

18. Einrichtung nach Anspruch 15, 16 oder 17, dadurch gekennzeichnet, daß der Meßaufnehmer ein Sensor für Chlorwasserstoff und oder Stickstoffmonoxid ist.

19. Einrichtung nach einem der Ansprüche 1 bis 14, dadurch gekennzeichnet, daß insbesondere zusätzlich als Meßaufnehmer ein Radiationsthermometer für die Temperatur des Organismus vorgesehen ist, das in seinem Ausgang den Temperaturwert als elektrisches Signal abgibt.

20. Einrichtung nach Anspruch 19, daß das Radiationsthermometer als Meßorgan zur Messung der Temperatur des Organismus in einer Körperöffnung, insbesondere im im Ohr des Organismus ausgebildet ist.

21. Einrichtung nach einem der vorhergehenden Ansprüche dadurch gekennzeichnet, daß sie mit einem Gerät zur Beaufschlagung eines Organismus mit pulsierenden Magnetfeldern eine Geräteeinheit bildet.

22. Einrichtung nach Anspruch 21, dadurch gekennzeichnet, daß bei einer Auswerteschaltung mit einem weiteren Speicher eine Sperreinrichtung vorgesehen ist, die nur bei angeschaltetem weiteren Speicher die Aktivierung des Geräts zuläßt.

23. Einrichtung nach Anspruch 20, 21 oder 22, dadurch gekennzeichnet, daß eine Sperreinrichtung vorgesehen ist, die eine Aktivierung des Geräts nur am späten Vormittag und/oder am späten Nachmittag zuläßt.

## Claims

1. Device for determining the effect of pulsed primary magnetic fields on an organism, in which an evaluation circuit is provided for the signals derived from the organism by means of a measurement pick-up designed, in a preferred embodiment, as a measurement coil for secondary field signals, characterised in that the evaluation circuit has a storage device, that the storage device has a storage medium to which is allocated a control device configured so that several consecutive individual signals are written into the storage medium in such a way that they are combined into a cumulative signal in the storage medium, and that this cumulative signal consisting of several individual signals is the output signal of the evaluation circuit.

2. The device in accordance with Claim 1, characterised in that a suppression circuit is provided in the evaluation circuit between the measurement pick-up for the individual signals and the storage medium, and that the suppression circuit has a control unit which only permits individual signals to be written in at periods of rest following a primary magnetic field pulse.

3. The device in accordance with Claim 1 or 2, characterised in that the control unit of the storage medium is designed in such a way that the cumulative signal is the mean value of several individual values.

4. The device in accordance with Claim 3, characterised in that the circuit for determining the average value takes the form of a correlator which performs algebraic addition on the individual values and geometric addition on the disruptive signals.

5. The device in accordance with one of Claims 1 to 4, characterised in that the evaluation circuit, the storage medium and the control unit are designed in such a way that the amplitude value is determined from individual signals.

6. The device in accordance with one of Claims 1 to 4, characterised in that the evaluation circuit, the storage medium and the control unit are designed in such a way that the energy content is determined from individual signals.

7. The device in accordance with one of Claims 1 to 6, characterised in that the evaluation circuit, the storage medium and the control unit are designed in such a way that differential signals are formed from individual signals and stored.

8. The device in accordance with one of Claims 1 to 7, characterised in that the evaluation circuit and the control unit of the storage medium are designed in such a way that they feed to a further storage medium the cumulative signals occurring at the beginning and/or the end of each occasion when the organism is subjected to pulsed magnetic fields, with these cumulative signals particularly taking the form of differential signals.

9. The device in accordance with Claims 8, characterised in that the further storage medium is a storage medium which can be separated from the device, and is in particular in the form of a memory chip card.

10. The device in accordance with one of Claims 1 to 9, characterised in that the storage medium is divided into several areas in accordance with prior art, one of which is used for storing values which have been determined, another of which is used for storing treatment data, and another is intended as an area with restricted access for storing personal data related to the organism.

11. The device in accordance with one of Claims 1 to 10, characterised in that to ensure that the individual signals are comparable, a circuit is provided which derives a trigger signal in response to the movements of the organism, that the evaluation circuit has a trigger element to which the trigger signal is sent and which only sets the evaluation circuit to operational status at specific times as determined by the trigger signal.

12. The device in accordance with one of Claims 1 to 11, characterised in that in order to ensure that the individual signals in the storage medium are comparable, in a measurement pick-up designed as a measurement coil and during measurements of signals from the secondary magnetic field, the measurement coil is firmly attached to the organism.

13. The device in accordance with one of Claims 1 to 12, characterised in that in order to increase the signal-to-noise ratio in the evaluation circuit, in a measurement pick-up designed as a measurement coil and during measurements of signals from the secondary magnetic field, another coil is allocated to the measurement coil which serves to pick-up the secondary field signal from the organism and which is located away from the measurement coil and positioned so that it is only principally affected by the disruptive magnetic fields which also affect the measurement coil, and so that both coils are connected to the evaluation circuit input using an electrical differential circuit with respect to their output signals which are derived from the disruptive fields.

14. The device in accordance with one of Claims 1 to 13, characterised in that in order to increase the signal-to-noise ratio in the evaluation circuit, in a measurement pick-up designed as a measurement coil and during measurements of signals from the secondary magnetic field, the measurement coil is shielded against external disruptive magnetic fields.

15. The device in accordance with one of Claims 1 to 14, characterised in that in particular additionally as a measurement pick-up, an electrochemical recording device is provided for gases evolved by the organism or for liquids specific to the organism.

16. The device in accordance with Claim 15, characterised in that in the case of a measurement pick-up for gases, this is incorporated within a breathing mask.

17. The device in accordance with Claim 16, characterised in that the breathing mask has three connections, one of which is for the air supply at overpressure, another of which is for accommodating the gas sensor, and another of which contains the pressure regulator which ensures that the mask largely retains a constant overpressure for the passage of gas from the gas intake through the gas sensor and outwards into the space beyond the breathing mask.

18. The device in accordance with Claim 15, 16 or 17, characterised in that the measurement pick-up is a sensor device for hydrogen chloride and/or nitrogen monoxide.

19. The device in accordance with one of Claims 1 to 14, characterised in that in particular additionally as a measurement pick-up, a radiation thermometer is provided for measuring the temperature of the organism, with the radiation thermometer giving the temperature value as an electrical signal at its output.

20. The device in accordance with Claim 19, characterised in that the radiation thermometer is a measurement organ for measuring the temperature of the organism in one of its bodily openings, in particular in the ear of the organism.

21. The device in accordance with one of the above Claims, characterised in that the device forms a single device unit together with a device for subjecting an organism to pulsating magnetic fields.

22. The device in accordance with Claim 21, characterised in that a blocking device is provided in an evaluation circuit with an additional storage medium, whereby the blocking device only permits activation of the device when the additional storage medium is switched on.

23. The device in accordance with Claim 20, 21 or 22, characterised in that a blocking device is provided which only permits the device to be activated during the late morning and/or late afternoon.

## Revendications

1. Dispositif pour la détermination de l'effet exercé par des champs magnétiques primaires pulsés sur un organisme, dans lequel il est prévu un circuit d'évaluation des signaux transmis par l'organisme par l'intermédiaire d'un capteur de mesure conçu de préférence sous la forme d'une bobine détectrice de signaux de champs secondaires,
**caractérisé en ce que**
le circuit d'évaluation comprend un équipement de mémorisation, que l'équipement de mémorisation comprend une mémoire à laquelle il est assigné une équipement de mémorisation de telle forme, que l'enregistrement de plusieurs signaux individuels consécutifs dans la mémoire se fait de sorte que, dans la mémoire, il en est formé un signal cumulé et que ce signal cumulé consistant de plusieurs signaux individuels est prévu en tant que signal de sortie du circuit d'évaluation.

2. Dispositif d'après la revendication 1
**caractérisé en ce que**
dans le circuit d'évaluation il est prévu, entre le capteur de mesure pour les signaux individuels et la mémoire, un circuit de suppression et que, pour ce circuit de suppression, il est prévu une commande qui ne permet l'enregistrement de signaux individuels qu'au cours des temps de repos suivant une impulsion de champ magnétique primaire.

3. Dispositif d'après la revendication 1 ou 2,
**caractérisé en ce que**
la commande de la mémoire est conçue telle que le signal cumulé constitue la moyenne de plusieurs signaux individuels.

4. Dispositif d'après la revendication 3,
**caractérisé en ce que**
le circuit prévu pour la formation de la moyenne est conçu sous la forme d'un corrélateur qui exécute une addition algébrique pour les valeurs individuelles et une addition géométrique pour les signaux parasites.

5. Dispositif d'après une des revendications 1 à 4,
**caractérisé en ce que**
le circuit d'évaluation, la mémoire et la commande sont conçus de sorte qu'il est déterminé la valeur d'amplitude des signaux individuels.

6. Dispositif d'après une des revendications 1 à 4,
**caractérisé en ce que**
le circuit d'évaluation, la mémoire et la commande sont conçus de sorte qu'il est déterminé le contenu énergétique des signaux individuels.

7. Dispositif d'après une des revendications 1 à 6,
**caractérisé en ce que**
le circuit d'évaluation, la mémoire et la commande sont conçus de sorte qu'il est formé et mémorisé des signaux différentiels à partir des signaux individuels.

8. Dispositif d'après une des revendications 1 à 7,
**caractérisé en ce que**
le circuit d'évaluation et la commande de la mémoire sont conçus de sorte qu'ils transmettent à une mémoire supplémentaire les signaux cumulés, notamment sous la forme de signaux différentiels, qui se produisent respectivement au début et/ou à la fin de la soumission d'un organisme à des champs magnétiques pulsés.

9. Dispositif d'après la revendication 8,
**caractérisé en ce que**
la mémoire supplémentaire est une mémoire séparable de l'équipement, notamment sous la forme d'une carte puce mémoire.

10. Dispositif d'après une des revendications 1 à 9,
**caractérisé en ce que**
la mémoire est subdivisée de la façon connue en plusieurs sections, dont une est prévue pour la mémorisation des valeurs déterminées, une pour la mémorisation des données traitées et une, conçue en tant que mémoire sans accès, destinée à des données personnelles relatives à l'organisme.

11. Dispositif d'après une des revendications 1 à 10,
**caractérisé en ce que**
pour la protection de signaux individuels comparables, il est prévu un circuit pour la dérivation d'un signal de déclenchement des mouvements de l'organisme, que le circuit d'évaluation comporte une section de déclenchement qui reçoit le signal de déclenchement et que le circuit de déclenchement n'effectue les commutations qu'aux moments déterminés par le signal de déclenchement.

12. Dispositif d'après une des revendications 1 à 11,
**caractérisé en ce que**
dans le cas d'un capteur de mesure conçu sous la forme d'une bobine détectrice et de mesure des signaux du champ magnétique secondaire, il est prévu pour la bobine détectrice un ancrage fixe sur l'organisme, qui sert à la protection de signaux individuels comparables dans la mémoire.

13. Dispositif d'après une des revendications 1 à 12,
**caractérisé en ce que**
dans le cas d'un capteur de mesure conçu sous la forme d'une bobine détectrice et de mesure des signaux du champ magnétique secondaire, il est assigné à la bobine détectrice servant à la réception du signal de champ secondaire provenant de l'organisme, une bobine détectrice supplémentaire destinée à l'augmentation du rapport signal/bruit dans le circuit d'évaluation, de sorte qu'elle n'est traversée essentiellement que par les champs magnétiques parasites traversant également la bobine détectrice et que, pour ce qui est leurs signaux de sortie provenant des champs parasites, les deux bobines sont liées électriquement en connexion différentielle avec l'entrée du circuit d'évaluation.

14. Dispositif d'après une des revendications 1 à 13,
**caractérisé en ce que**
dans le cas d'un capteur de mesure conçu sous la forme d'une bobine détectrice et de mesure des signaux du champ magnétique secondaire, la bobine détectrice est blindée contre des champs magnétiques parasites extérieures en vue de l'augmentation du rapport signal/bruit dans le circuit d'évaluation.

15. Dispositif d'après une des revendications 1 à 14,
**caractérisé en ce que**,
notamment en tant que capteur de mesure supplémentaire, il est prévu un capteur électrochimique pour les gaz ou les liquides organiques spécifiques émanant de l'organisme.

16. Dispositif d'après la revendication 15,
**caractérisé en ce que**
dans le cas d'un capteur de mesure pour des gaz, celui-ci est intégré dans un respirateur.

17. Dispositif d'après la revendication 16,
**caractérisé en ce que**
le respirateur est muni de trois raccords dont un est prévu pour l'arrivée d'air avec surpression, un pour le logement du capteur de gaz et un pour un régulateur de surpression assurant une surpression largement constante pour le passage des gaz de l'entrée, par l'intermédiaire du capteur, à la chambre à l'extérieur du respirateur.

18. Dispositif d'après la revendication 15,16 ou 17,
**caractérisé en ce que**
le capteur de mesure est un capteur pour gaz hydrochloriques ou pour monoxydes d'azote.

19. Dispositif d'après une des revendications 1 à 14,
**caractérisé en ce que**,
notamment en tant que capteur de mesure supplémentaire, il est prévu pour la température de l'organisme un thermomètre différentiel dont la sortie transmet la valeur de température sous forme d'un signal électrique.

20. Dispositif d'après la revendication 19,
**caractérisé en ce que**
le thermomètre différentiel est conçu sous la forme d'un organe de mesure de la température de l'organisme dans une ouverture du corps, notamment dans l'oreille de l'organisme.

21. Dispositif d'après une des revendications précédentes,
**caractérisé en ce**
qu'il forme une unité avec l'appareil destiné à soumettre un organisme aux champs magnétiques pulsés.

22. Dispositif d'après la revendication 21,
**caractérisé en ce que**
dans le cas d'un circuit d'évaluation avec une mémoire supplémentaire, il est prévu un dispositif de barrage ne permettant l'activation du dispositif que du moment que la mémoire supplémentaire est activée elle-aussi.

23. Dispositif d'après la revendication 20, 21 ou 22,
**caractérisé en ce**
qu'il est prévu un dispositif de barrage ne permettant l'activation du dispositif que tard dans la matinée et/ou tard dans l'après-midi.
